# EUROPEAN PATENT APPLICATION

(11) **EP 4 147 662 A1**
(43) Date of publication of application: **15.03.2023**
(21) Application number: 22195688.1
(22) Date of filing: 14.09.2022
(51) Int. Cl.: A61B 34/20, A61B 34/30, A61B 90/30, A61B 90/00, G16H 40/63

(54) **AUTONOMOUS SURGICAL SENSORS AND COMMUNICATION NETWORK**

(30) Priority: 14.09.2021 US 202163244231 P; 29.07.2022 US 202217877867
(71) Applicant: Covidien LP, Mansfield, MA 02048 (US)
(72) Inventor: Komp, John W., Boulder, 80301 (US)
(74) Representative: Maschio, Antonio

(57) **Abstract**

Surgical systems and methods involve an instrumentation system disposed external to a body of a patient, sensors, which include a first antenna, configured to be placed within the body of the patient, and a surgical instrument including a second antenna disposed at a proximal portion of the surgical instrument. The surgical instrument communicates with the sensors via the first and second antennas. The surgical instrument also communicates messages with the instrumentation system when the surgical instrument is placed in the body of the patient, but the second antenna is disposed external to the body of the patient. The messages include sensor data from the sensors.

## Description

### CROSS-REFERENCE TO RELATED APPLICATIONS

This application claims the benefit of the filing date of provisional U.S. Application No. 63/244,231, filed September 14, 2021.

### FIELD

This disclosure is generally related to a communication network for minimally invasive surgery or robot-assisted surgery, in which surgical instruments and intrabody sensors are used.

### BACKGROUND

In minimally invasive surgery, small incisions are made in a patient and a surgical procedure is performed using surgical instruments including an endoscope placed through the small incisions. Because the surgeon makes small incisions instead of a large opening, the patient likely has a reduced recovery time and less pain than traditional open surgery. In some cases, the surgeon may want more information regarding the surgical site than can be provided by the video from the endoscope. Wired or wireless sensors may be placed at the surgical site to obtain more information regarding the surgical site. However, the wires of the wired sensors are inconvenient to manage and may obstruct the view of the surgical site. Also, the wireless sensors may not be able to communicate with an instrumentation system located external to the body of the patient.

### SUMMARY

This disclosure generally relates to systems and methods for adding sensors to existing surgical tools and sensors without redesign, a surgical site communication network, and a secure method for creating the surgical site communication network.

In one aspect, this disclosure provides a surgical system. The surgical system includes an instrumentation system, sensors, and a surgical instrument. The instrumentation system is disposed external to a body of a patient. The sensors are placed within the body of the patient. Each sensor includes a first communication device including a first antenna. The surgical instrument includes a second communication device. The second communication device includes a second antenna disposed at a proximal portion of the surgical instrument. The second communication device communicates with the sensors via the first and second antennas. The second communication device communicates messages to or from the instrumentation system when the surgical instrument is placed in the body of the patient. The messages include sensor data from the sensors.

Implementations may include one or more of the following features. The second antenna may act as a master antenna. The sensors may communicate with each other via the first antennas of the sensors.

The surgical instrument may be a laparoscope. The second communication device may include a microcontroller that configures the sensors to only communicate with the second communication device.

The surgical system may include a second surgical instrument including a third communication device including a third antenna and a second microcontroller. The second microcontroller may be configured to communicate with the second communication device and the sensors via the first, second, and third antennas.

The surgical instrument may include a microcontroller configured to build a mesh network including the surgical instrument and the sensors.

The instrumentation system may include a microcontroller configured to detect a failure event associated with the surgical instrument or one of the sensors; and, in response to detecting the failure event associated with the surgical instrument or one of the sensors, removing the surgical instrument or one of the sensors from the mesh network. The failure event may include movement external to the body of the patient. The failure event may include failure of a battery of at least one of the sensors.

In another aspect, this disclosure provides a surgical communication method. The method includes receiving, through a first antenna of a surgical instrument having a portion thereof disposed within a body of a patient, EM sensor signals from second antennas of sensors placed within the body of the patient. The method also includes transmitting, from the first antenna of the surgical instrument, an EM signal to an instrumentation system disposed external to the body of the patient.

Implementations may include one or more of the following features. The method may include building a mesh network of first communication devices of the sensors and a second communication device of the surgical instrument. The method may include detecting a termination event associated with the surgical instrument or one of the sensors; and, in response to detecting the termination event associated with the surgical instrument or one of the sensors, removing the second communication device of the surgical instrument or the first communication device of one of the sensors from the mesh network. Detecting the termination event may include detecting movement of one of the sensors to a location external to the body of the patient or detecting failure of a battery of at least one of the sensors.

The method may include identifying sensors within the body of the patient, detecting movement of the surgical instrument to a location external to the body of the patient, and, in response to detecting movement of the surgical instrument to a location external to the body of the patient, determining that not all sensors are at a location external to the body of the patient, and, in response to determining that not all sensors are at a location external to the body of the patient, generating a message indicating that not all sensors are at a location external to the body of the patient.

The method may include periodically receiving, at the first antenna of the surgical instrument, a low-power signal including identification and status information from the second antennas of the sensors. The method may include receiving, at the first antenna, low-power EM signals, which include secure keys, that cannot be detected outside of the body of the patient to initiate secure communication between the surgical instrument and the sensors.

In still another aspect, this disclosure provides a surgical communication method. The surgical communication method includes receiving, through a first antenna of a surgical instrument having a portion thereof disposed within the body of the patient, low-power EM signals, which include secure keys that cannot be detected outside of the body of the patient to initiate secure communication between the surgical instrument and sensors placed within the body of the patient. The method further includes transmitting, from the first antenna of the surgical instrument, an EM signal to an instrumentation system disposed external to the body of the patient. The EM signal may include EM sensor signals received by the first antenna from second antennas of the sensors.

Implementations may include one or more of the following features. The method may include identifying sensors within the body of the patient; detecting movement of the surgical instrument to a location external to the body of the patient; in response to detecting movement of the surgical instrument to a location external to the body of the patient, determining that not all sensors are at a location external to the body of the patient; and in response to determining that not all sensors are at a location external to the body of the patient, generating a message indicating that not all sensors are at a location external to the body of the patient. The method may include building a mesh network of the surgical instrument and the sensors.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a diagram that illustrates a surgical system of the disclosure;
FIG. 2 is a block diagram that illustrates a surgical communication system of the disclosure; and
FIGS. 3 and 4 are flow charts that illustrate methods of operating the surgical communication system of FIG. 2.

### DETAILED DESCRIPTION

The systems and methods of this disclosure involve small, battery-powered sensors, which may include a detector and an optional stimulation source, and which may be placed individually at a surgical site, such as a surgical cavity. The systems and methods of this disclosure also involve a communication network to allow for intrabody communication among the sensors and surgical instruments, and communication from the sensors to a system outside the body of the patient.

In minimally invasive surgery, including robotic- and video-assisted surgery, there is a desire to increase the amount of information extracted from the surgical site to build a more complete understanding of the surgical site, which may improve the surgeon's ability to perform a surgical procedure precisely and efficiently. To this end, one or more sensors are deployed in the surgical cavity or field. The sensors may include a measurement device or an imagery system. Also, the sensors may be standalone sensors or may be integrated into a surgical tool. In one aspect, at least one of the standalone sensors may be a small camera with robotic legs.

According to aspects of this disclosure, sensor data, e.g., measurement and/or imagery data, is collected from the sensors and transmitted to an external processing system at a location external to the body of the patient. The external processing system, e.g., a workstation, may then process the sensor and display the sensor data to the surgeon. One or more surgical instruments associated with one or more sensors may route the collected sensor data through the surgical instruments using communication wires coupled to a proximal portion of the surgical instrument's handle. A cable may, in turn, be connected between the handle of the surgical instrument and the external processing system.

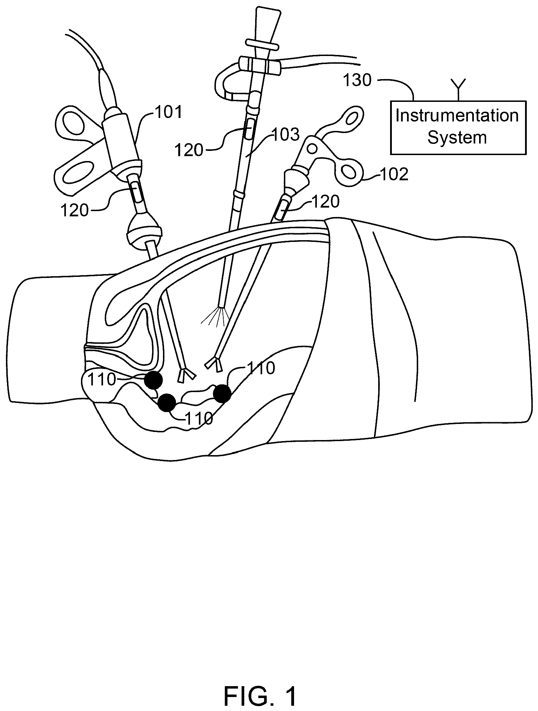

The above-described configuration may increase the number of cables outside the patient, which may increase tripping risks, may lead to connection confusion, and may result in physical tangling. An alternative configuration includes replacing the cable with a radio or electromagnetic (EM) communication system. With reference to FIG. 1, the EM communication system includes communication devices 120, which are located at the proximal end portion of the surgical instruments 101-103 and which are located outside of the body of the patient. The communication devices may include microcontrollers or any suitable processors for implementing the features and/or functions of the communication devices 120. The one or more sensors 110 wirelessly send the data (e.g., using a high frequency protocol, such as Bluetooth@, amongst others) to a common collection device, e.g., an instrumentation system 130, in a surgical suite. The instrumentation system 130 may be implemented by a console or a workstation. The one or more sensors 110 may include microcontrollers or any suitable processors for implementing the features and/or functions of the one or more sensors 110. The EM communication system of FIG. 1 does not require routing wires from distally mounted sensors to a proximal end portion of the surgical instruments 101-103, where data can then be sent through wires or radio to a data processing system. As can be appreciated, routing wires in the surgical tools may add complexity, cost, and constraints on performance.

The one or more sensors 110 may include cameras, stimulation devices, and other sensors (which may be implemented in independent instruments) that are selfcontained and battery-powered with no external connection. The one or more sensors 110 may be placed or attached to anatomy within the surgical cavity. They may also be placed temporarily or permanently on the distal end portion of a surgical instrument. The one or more sensors 110 may include a video camera, which gives an additional view of the surgical site, or a light source, which is used for surgical illumination, and which may transmit light through anatomy to a detector (e.g., a pulse-oximeter). The one or more sensors 110 may include an ultrasound source or receiver, or a chemical sensor. In some aspects, the one or more sensors 110 may include a sensor that incorporates two or more different types of sensors.

The one or more sensors 110 may rely on a radio system for communication. High-frequency, high-bandwidth systems operate in frequency bands, such as the 2.4GHz band used by Bluetooth@, and do not penetrate far through the body of the patient when operating at the low output power of battery-powered devices. A low-power network with a master antenna of a surgical instrument enables the EM communication system of this disclosure.

Although generally described as being associated with discrete surgical instruments, it is envisioned that one or more of the surgical instruments 101-103 described herein may be associated with robotic arms of a robotic surgical system or the like.

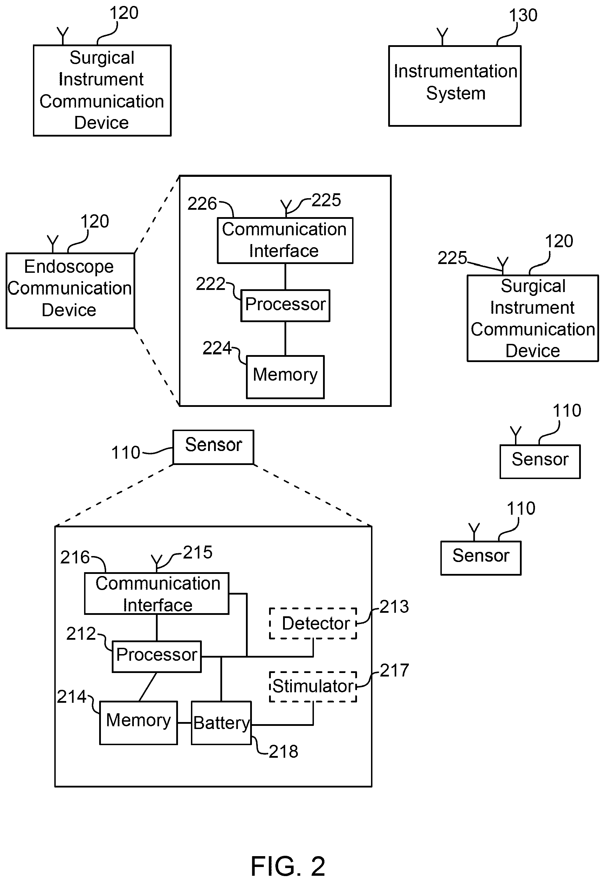

As shown in FIGS. 1 and 2, surgical instrument communication devices 120, which include master antennas 225 (FIG. 2), are attached to or incorporated into surgical instruments 101-103 that have a proximal end or cable that exits the body of the patient. One or more of the surgical instrument communication devices 120 is used to couple the network of sensors 110 in the surgical cavity to an instrumentation system 130 external to the body of the patient. In some aspects, the surgical instrument communication device 120 may be integrated into a laparoscope 103. Independent surgical instruments 101-103 in the surgical cavity may build a mesh network, in which one or more of the surgical instrument communication devices 120 is a peer device. Each sensor 110 may be configured such that each sensor 110 can only directly communicate with one of the surgical instrument communication devices 120 via the master antenna 225 (FIG. 2). The independent surgical instruments may share information, such as information received from one or more of the sensors, between each other and devices located a short distance from the patient, including, for example, the instrumentation system 130.

The mesh network may be built by configuring a first sensor 110 as a repeater for a second sensor 110 that may be unable to communicate reliably with a surgical instrument communication device 120. A surgical instrument communication device 120 may also request a first sensor 110 to take over direct communication with a second sensor 110 when the current direct connection with the surgical instrument communication device 120 is unreliable.

Communication reliability may be achieved by configuring each sensor 110 and each surgical instrument communication device 120 to monitor the signal strength of all devices that each sensor 110 and each surgical instrument communication device 120 can see. If a surgical instrument communication device 120 determines that the signal strength of one or more of the sensors 110 is low or below a threshold, the surgical instrument communication device 120 can request other 110s to return their signal strengths to the sensors 110. If one of those is greater than the surgical instrument communication device 120, that sensor 110 becomes a repeater for the low signal strength. If none are greater, the surgical instrument communication device 120 may send a signal strength issue message to 130 for consideration.

With continued reference to FIG. 2, a detailed view of the surgical instrument communication devices 120 is illustrated. Each of the surgical instrument communication devices 120 is substantially similar, and therefore, one surgical instrument communication device 120 will be described in detail in the interest of brevity. The surgical instrument communication device 120 includes a memory 224, a processor 222 that is operably coupled to the memory 224, and a communication interface 226 that is operably coupled to the processor 222. The communication interface 226 includes a master antenna 225 that is configured to wirelessly transmit and receive data amongst other surgical instrument communication devices 120, the various sensors 110, and the instrumentation system 130. In aspects, the communication interface 226 may include two or more antennas. A first antenna may be disposed on or in a proximal portion of a surgical instrument, such that the first antenna is outside of a patient's body and is configured to communicate with the instrumentation system 130. And a second antenna may be disposed on or in a distal portion of the surgical instrument, such that the second antenna is inside of the patient's body and is configured to communicate with one or more of the sensors using, for example, low-power communication. It is envisioned that the master antenna 225 of the communication interface 226 may communicate with any suitable device capable of transmitting and/or receiving wireless data, depending upon the design needs of the EM communication system.

It is also envisioned that the master antenna 225 may operate according to a suitable master/slave relationship. For example, the master antenna 225 and associated electronics may operate as a communication hub that supervises or controls communications with slave devices. In some situations, or during certain periods, the master antenna 225 and associated electronics may also operate as a slave device with respect to another master device, e.g., the instrumentation system 130.

Alternatively, the surgical instrument communication devices 120 may include two antennas. One antenna may be disposed at a distal portion of the surgical instrument and another antenna may be disposed at a proximal portion of the surgical instrument. The distal antenna may communicate with one or more of the sensors 110, e.g., receive sensor data from the one or more sensors 110, and the proximal antenna may communicate with the instrumentation system 130, e.g., transmit the received sensor data to the instrumentation system 130. The communication via the proximal and distal antennas may be performed at different power levels and data rates. For example, the communication via the distal antenna may be performed at a higher power level and higher data rate than the communication via the proximal antenna. In another alternative, two surgical instrument communication devices 120 may be disposed in or on a surgical instrument: a first surgical instrument communication device 120 disposed in or on a distal portion of the surgical instrument and a second surgical instrument communication device 120 disposed in or on a proximal portion of the surgical instrument. The two surgical instrument communication devices 120 may communicate with each other via wireless or wired communication.

Continuing with FIG. 2, a detailed view of the one or more sensors 110 is illustrated. Each of the one or more sensors 110 may be substantially similar, and therefore, one sensor 110 will be described in detail in the interest of brevity. The sensor 110 includes a processor 212, a memory 214 that is operably coupled to the processor 212, a communication interface 216 that is operably coupled to the processor 212, and an energy storage device 218 that is operably coupled to each of the processor 212, and communication interface 216. It is contemplated that the energy storage device 218 may be any suitable energy storage device, such as a battery, and may be recharged using any suitable technique, such as wirelessly, through a wired connection, etc. For example, the energy storage device 218 may be recharged wirelessly though energy scavenging, such as radio frequency (RF) power harvesting. The communication interface 216 includes an antenna 215 that is configured to wirelessly transmit and receive data amongst one or more other sensors 110, one or more surgical instrument communication devices 120, the instrumentation system 130. It is envisioned that the antenna 215 of the communication interface 216 may communicate with any suitable device capable of transmitting and/or receiving wireless data, depending upon the design needs of the system.

As described hereinabove, the sensor 110 may include a camera (e.g., video, still, etc.), which gives an additional view of the surgical site, a chemical sensor, a light source, which is used for surgical illumination or as part of a sensor and which transmits light through anatomy to a detector 213 (e.g., a pulse-oximeter), an ultrasound source or receiver, and a stimulator 217, amongst others. In non-limiting aspects, the sensor 110 includes a detector 213 or a stimulator 217. It is envisioned that the various sensors 110 may include the same or similar components, depending upon the design needs of the system. In this manner, one sensor 110 may include a video camera to provide additional views of the surgical site, a second sensor 110 may include a light source to illuminate a portion of the surgical site, and a third sensor 110 may include a detector 213 to detect light to act as a pulse oximeter or the like. As can be appreciated, any combination of sensors 110 may be employed to best assist the clinician performing the surgical procedure.

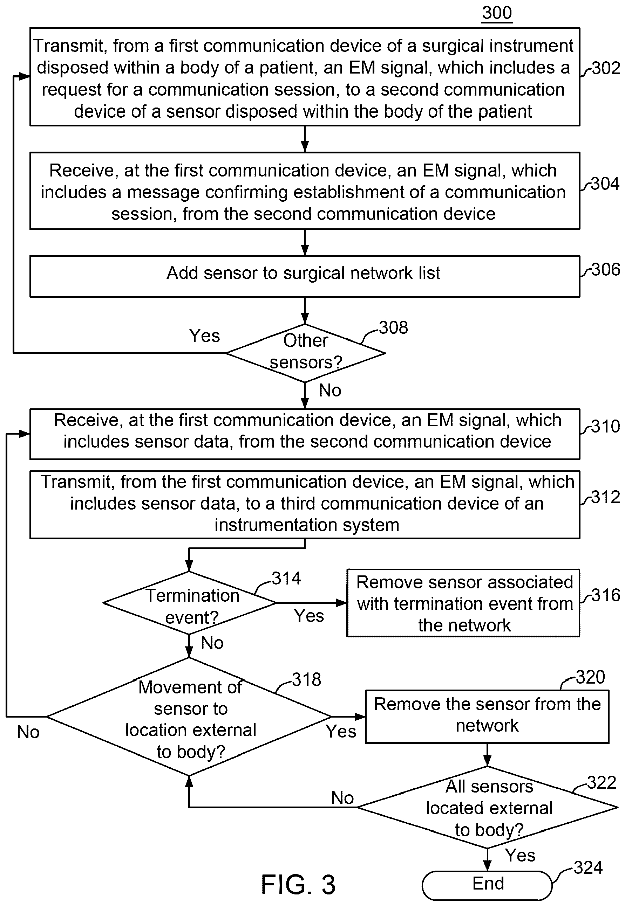

FIG. 3 illustrates an example of a method 300 executed by a communication device 120 of a surgical instrument 101. At block 302, a first communication device 120 of a surgical instrument 101 disposed within a body of a patient transmits an EM signal, which includes a request for a communication session, to a second communication device of a sensor 110 disposed within the body of the patient. At block 304, the first communication device 120 receives an EM signal, which includes a message confirming establishment of a communication session, from the second communication device of the sensor 110. At block 306, the sensor 110 is added to a surgical network list stored in the memory 224 of the first communication device 120. The first communication device 120 may also transmit a message to a third communication device, e.g., the instrumentation system 130 of FIGS. 1 and 2, requesting that the sensor 110 be added to a surgical network list stored in the memory (not shown) of the third communication device.

At block 308, the method 300 determines whether there are other sensors 110, which are disposed within the body of the patient, and which have not yet joined the surgical communication network. If there are other sensors 110, which are disposed within the body of the patient and which have not yet joined the surgical network, blocks 302-306 are repeated until all sensors 110 disposed within the body of the patient have joined the surgical network. If all sensors 110 have joined the surgical communication network, the first communication device 120 receives an EM signal, which includes sensor data, from the second communication device of the sensors 110, at block 310.

At block 312, the first communication device 120 transmits an EM signal, which includes sensor data acquired by a sensor 110, to a third communication device of the instrumentation system 130, which is disposed external to the body of the patient. At block 314, the method 300 determines whether a termination event has occurred. The termination event may include a sensor battery failure. If there is a termination event, the sensor 110 associated with the termination event is removed from the network at block 316. If a termination event has not occurred, the method 300 determines, at block 318, whether there is movement of a sensor 110 to a location external to the body of the patient. In aspects where the second communication device 120 includes two antennas, the movement of the sensor 110 external to the body may be detected by the first communication device's 110 communication signal moving from the distal antenna of the second communication device 120 (that is, the antenna in or on the distal portion of the surgical instrument that is inside the patient's body) to the proximal antenna of the communication device 120 (that is, the antenna in or on the proximal portion of the surgical instrument that is outside the patient's body). If there is movement of a sensor 110 to a location external to the body of the patient, for example, when a surgical procedure is completed, the sensor 110 is removed from the network at block 320. If there is no movement of a sensor 110 to a location external to the body of the patient, the method 300 repeats block 310 and subsequent blocks.

At block 322, the method 300 determines whether all sensors 110 are located external to the body. If the method 300 determines that all sensors 110 are located external to the body, the method 300 ends at block 324. If the method 300 determines that all sensors 110 are not located external to the body, the method 300 repeats block 318 until the method 300 determines that all sensors 110 are located external to the body of the patient. As can be appreciated, identifying whether all sensors 110 have been removed from the body of the patient helps to ensure that no foreign bodies are left within the patient after the completion of the surgical procedure, thereby increasing patient safety.

Data security may be implemented by a method in which secure keys are passed between the communication devices 120 of the surgical instruments 101-103 and the communication interfaces 216 of the sensors 110, e.g., master communication devices of the surgical instruments 101-103 and slave communication devices of the sensors 110, using low-power messaging, which cannot be detected outside of the body of the patient. The method may include a layer of security on top of the standard Bluetooth@ low energy (LE) security through the exchange and validation of additional vendor signed keys.

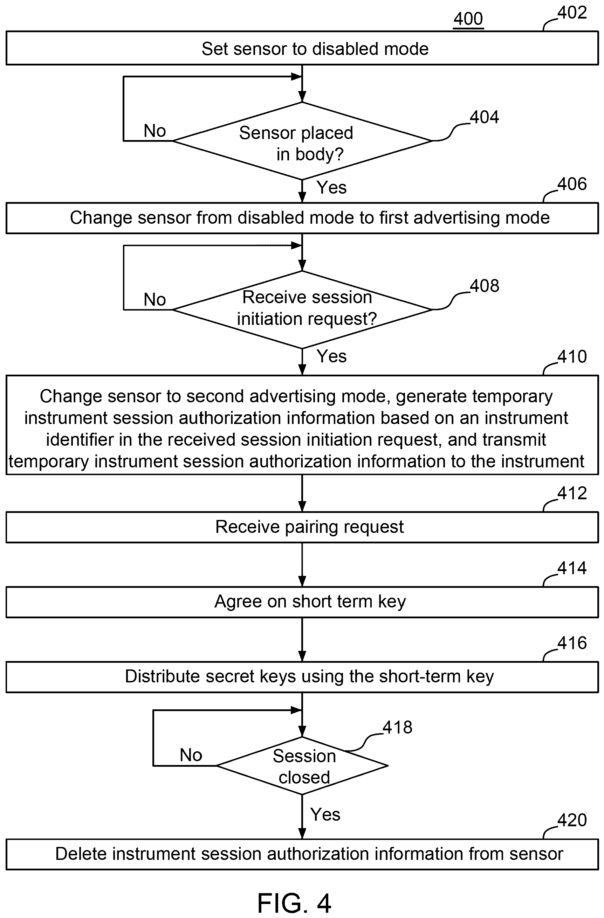

FIG. 4 illustrates an example a method 400 of establishing a secure connection between a communication device 120 of a surgical instrument 101 and a communication device of a sensor 110. At block 402, a communication device of a sensor 110 is initially set to operate in a disabled mode, in which telemetry communication is prevented by the communication device of the sensor 110 from using a Bluetooth^{®} LE protocol. At block 404, the method 400 determines whether the sensor 110 is placed in the body of a patient. Block 404 may be implemented by determining the location of the sensor 110 with respect to the body of the patient using the antenna 215 of the sensor 110 and an EM field generated by an EM field generator (not shown). If the method 400 determines that the sensor 110 is placed in the body of the patient, the sensor 110 is changed at block 406 from the disabled mode to a first advertising mode, in which first advertisement data packets are transmitted according to the Bluetooth@ LE protocol at a first rate. If the method 400 determines that the sensor 110 has not been placed within the body of the patient, the method 400 repeats block 404 until the method 400 determines that the sensor 110 is located within the body of the patient.

At block 408, the method 400 determines whether a surgical instrument session initiation request is received from a communication device 120 of a surgical instrument 101 via the Bluetooth@ LE protocol while the communication device of the sensor 110 is operating in the first advertising mode or in the disabled mode. The surgical instrument session initiation request may include an identifier for the surgical instrument 101. If a surgical instrument session initiation request is received from the communication device 120 of the surgical instrument 101 via the Bluetooth@ LE protocol, the communication device of the sensor 110 transitions, at block 410, from operating in the first advertising mode (or the disabled mode) to operating in a second advertising mode, which includes transmitting second advertisement data packets according to the Bluetooth@ LE telemetry protocol at a second rate. If the method 300 determines that no surgical instrument session request has been received, the method 400 repeats block 408 until the method 400 determines that a surgical instrument session initiation request has been received from the communication device 120 of the surgical instrument 101.

At block 410, the communication device of the sensor 110 further generates temporary surgical instrument session authorization information based on a surgical instrument identifier in the received surgical session initiation request. The authorization information may include a unique session identifier and at least one unique session key. The session key may be a cryptographic key signed by the manufacturer and installed at the manufacturer's facility. The cryptographic key may be locally validated by the second communication device 120, by the instrumentation system 130, or remotely through a connection from the instrumentation system 130 to an external system (e.g., a security server) where counterfeit or expired keys may be denied. The sensor 110 may also validate the cryptographic key from the second communication device 120 to make sure that the second communication device 120 is a valid receiver since the sensor 110 does not have the ability or advantage of reaching back to the security server. The sensor 110 may validate the cryptographic key from the second communication device 120 by comparing the chain of authority in the second communication device's 120 cryptographic key compared with the sensor's 110 cryptographic key. The authorization information is employed to facilitate establishment of a telemetry session between the communication device of the sensor 110 and the communication device 120 of the surgical instrument 101. At block 410, the communication interface 216 of the sensor 110 further transmits temporary surgical instrument session authorization information to the communication device 120 of the surgical instrument 101.

In some aspects, in response to receiving a surgical instrument session initiation request, the communication device of the sensor 110 generates unique time sensitive authorization information that is required for the establishment of the requested surgical instrument session between the communication device of the sensor 110 and the communication device 120 of the surgical instrument 101.

Sensitive authorization information may be employed to establish the currently requested surgical instrument session. For example, after a surgical instrument session established between the communication interface 216 of the sensor 110 and the surgical instrument 101 using the authorization information is closed, the sensor 110 clears the authorization information from memory 214 (e.g., deletes the authorization information or treats the authorization information as expired). In addition, if the communication interface 216 of the sensor 110 and the surgical instrument 101 fail to establish a surgical instrument session using the authorization information within a defined window of time (e.g., the advertisement period), the communication device of the sensor 110 may clear the authorization information from memory 214 (e.g., delete the authorization information or otherwise treat the authorization information as expired). As a result, in order for the same communication device 120 of the surgical instrument 101 to establish a new surgical instrument session with the communication device of the sensor 110, in some aspects, the communication device 120 of the surgical instrument 101 transmits a new surgical instrument session initiation request to the communication device of the sensor 110 and the communication device of the sensor 110 generates new authorization information.

In some aspects, a timeout is initiated after a communication session is established. Normal communication between sensor 110 and second communication device 120 continues during the timeout, though at a low rate if needed (e.g., once every 1-5 seconds) as a communication heartbeat to assure the communication session is still valid. Otherwise, the communication session is dropped and the cryptographic key invalidated. This feature mitigates a replay attack.

In one example, the time sensitive authorization information includes a dynamically generated unique session identifier including random numbers (e.g., a universal unique identifier (UUID) or the like). The time sensitive authorization information also includes the cryptographic keys, which may be one or more dynamically generated unique session keys (e.g., an advanced encryption standard (AES) key or the like). For example, the communication device of the sensor 110 can generate a unique application layer encryption key (e.g., a 128 bit application layer encryption key) and unique link layer encryption key (e.g., a 128 bit link layer encryption key). The one or more unique session keys may be employed by the communication device of the sensor 110 and the communication device 120 of the surgical instrument 101 to encrypt and decrypt information communicated between the respective communication devices \during the surgical instrument session. The communication device of the sensor 110 may be configured to generate the unique time sensitive authorization information (e.g., the unique session identifier and the one or more session keys) in response to receiving the surgical instrument session initiation request. Accordingly, the authorization information is not previously known or available to any device, including the communication device of the sensor 110 and the communication device 120 of the surgical instrument 101.

The communication device of the sensor 110 may be further configured to transmit the dynamically-generated, time-sensitive authorization information to the communication device 120 of the surgical instrument 101 via an information signal formatted and transmitted to the communication device 120 of the surgical instrument 101 using the second telemetry communication protocol. For example, in an aspect in which the second telemetry communication protocol includes an induction-based protocol, the sensor 110 can be configured to send the authorization information to the clinician device using an induction-based telemetry communication signal. After the sensor 110 sends the authorization information to the surgical instrument 101 using the second telemetry communication protocol, the sensor 110 can begin operating in the second advertising mode 204. In one or more implementations, while operating in the second advertising mode, the communication device of the sensor 110 may generate and transmit one or more advertisement data packets that indicate the availability of the communication device of the sensor 110 to establish a telemetry session using the first telemetry communication protocol. The one or more advertisement data packets respectively include the unique session identifier (e.g., a UUID) generated by the communication device of the sensor 110 in response to reception of the clinician session request and provided to the surgical instrument 101 using the second telemetry communication protocol.

At block 412, a pairing request is received. At block 414, the communication devices of the surgical instrument 101 and the sensor 110 agree on a short-term key and store the short-term key in the memory 224, 214 of the communication devices of the surgical instrument 101 and the sensor 110. At block 416, the short-term key is used to distribute secret keys to the communication device of the surgical instrument 101 and the sensor 110. At block 418, the method 400 determines whether the session between the communication devices of the surgical instrument 101 and the sensor 110 is closed. If the session is closed, the surgical instrument session authorization information is deleted from the sensor 110 at block 420. If the method 300 determines that the session has not been closed, the method 400 repeats block 418 until the method 400 determines that session has been closed.

Patient safety related to retained surgical instruments 101-103 may be implemented through an independent surgical instrument accounting system and method based on the ability to continually communicate with the communication devices of the surgical instruments 101-103 and the sensors 110. When a surgical instrument 101 or sensor 110 is extracted from the body of the patient, the surgical instrument 101 or sensor 110 leaves the surgical cavity network, which is implemented by removing the sensor 110 from the known implanted sensor list. Communication devices of the sensors 110 may also leave the network if their energy storage device 218 fails. Each communication device of the sensors 110 may include a power fail mode, which may be triggered before full exhaustion of the energy storage device 218, e.g., before the energy storage device 218 reaches 15% or another desired percentage of a full state of charge. The communication devices may transition to a ping mode that uses a low-power signal to send identification and status information periodically to conserve power until the sensor 110, and therefore, the communication device is extracted.

In aspects, when a sensor 110 or surgical instrument communication device 120 is to be or is being extracted from the body of a patient, e.g., a surgical cavity, and a sensor 110 or a surgical instrument communication device 120 is currently located in the body, the sensor 110 or surgical instrument communication device 120 to be or being extracted may transfer its one or more communication channels with one or more of the sensors 110 with which the sensor 110 or surgical instrument communication device 120 to be or being extracted is communicating with to the one or more remaining surgical instrument communication devices 120. When a master communication device is extracted from the surgical field, e.g., a master surgical instrument communication device 120, the master communication device may look for all communication devices of the sensors 110 that were known to be within the body of the patient, e.g., a surgical cavity, and confirm that each communication device of the sensors 110 is active outside of the body of the patient. The master communication device may double check the accounting for all communication devices used during the procedure.

In some aspects, the sensors 110 may include three orthogonal coils and the system may include an electromagnetic navigation board (not shown), on which the patient lays. In this configuration, the electromagnetic navigation board generates an electromagnetic field, which is sensed by the three orthogonal coils. The magnitude of the sensed electromagnetic field may be used to determine the position of each sensor 110 in three-dimensional (3D) space. The sensors 110 may transmit the sensed electromagnetic field signals to the instrumentation system 130. Using the sensor location information, the console may mark each sensor's 110 location in a CT image of the patient. Or the instrumentation system 130 may build a 3D model of the CT images and show the location of each sensor 110 in the 3D model. Among other benefits, displaying the location of the sensors 110 to a user in the CT images or in the 3D model may mitigate accidental retention of the sensors 110 in the body of the patient.

If two or more of the sensor 110 are cameras, the locations of the sensors 110 may be used to stitch together the video feeds from the two or more sensors 110 into a combined view. The direction of the sensed electromagnetic field may be determined and used to determine the pointing direction of a cameras. The determined pointing direction may be used to show the viewpoint of each camera on the CT images or the 3D model. In some aspects, the system may include a multiple sensors 110 configured to function as a single sensor system where placement of each sensor 110 is critical to the functionality of the sensor system. The instrumentation system 130 may be configured to give instructions, e.g., through a display of the instrumentation system 130, on how to place and orient the sensors 110.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

The invention may be described by reference to the following numbered paragraphs:-
1. A surgical system, comprising:
   an instrumentation system disposed external to a body of a patient;
   sensors configured to be placed within the body of the patient, each sensor including a first communication device including a first antenna; and
   a surgical instrument including a second communication device including a second antenna disposed at a proximal portion of the surgical instrument, the second communication device configured to communicate with the sensors via the first and second antennas, the second communication device further configured to communicate messages to or from the instrumentation system when the surgical instrument is placed in the body of the patient, the messages including sensor data from the sensors.
2. The surgical system of paragraph 1, wherein the second antenna is configured to act as a master antenna.
3. The surgical system of paragraph 1, wherein the sensors are configured to communicate with each other via the first antennas of the sensors.
4. The surgical system of paragraph 1, wherein the surgical instrument is a laparoscope.
5. The surgical system of paragraph 1, wherein the second communication device includes a microcontroller that configures the sensors to only communicate with the second communication device.
6. The surgical system of paragraph 1, further comprising a second surgical instrument including a third communication device, which includes a third antenna and a second microcontroller, the second microcontroller configured to communicate with the second communication device and the sensors via- the first, second, and third antennas.
7. The surgical system of paragraph 1, wherein the surgical instrument includes a microcontroller configured to build a mesh network including the surgical instrument and the sensors.
8. The surgical system of paragraph 7, wherein the instrumentation system includes a microcontroller configured to:
   detect a failure event associated with the surgical instrument or one of the sensors; and
   in response to detecting the failure event associated with the surgical instrument or one of the sensors, removing the surgical instrument or one of the sensors from the mesh network.
9. The surgical system of paragraph 8, wherein the failure event includes movement external to the body of the patient.
10. The surgical system of paragraph 8, wherein the failure event includes failure of a battery of at least one of the sensors.
11. A surgical communication method, comprising:
   receiving, through a first antenna of a surgical instrument having a portion thereof disposed within a body of a patient, EM sensor signals from second antennas of sensors placed within the body of the patient; and
   transmitting, from the first antenna of the surgical instrument, an EM signal to an instrumentation system disposed external to the body of the patient.
12. The method of paragraph 11, further comprising building a mesh network of first communication devices of the sensors and a second communication device of the surgical instrument.
13. The method of paragraph 12, further comprising:
   detecting a termination event associated with the surgical instrument or one of the sensors; and
   in response to detecting the termination event associated with the surgical instrument or one of the sensors, removing the second communication device of the surgical instrument or the first communication device of one of the sensors from the mesh network.
14. The method of paragraph 13, wherein detecting the termination event includes detecting movement of one of the sensors to a location external to the body of the patient or detecting failure of a battery of at least one of the sensors.
15. The method of paragraph 11, further comprising:
   identifying sensors within the body of the patient;
   detecting movement of the surgical instrument to a location external to the body of the patient;
   in response to detecting movement of the surgical instrument to a location external to the body of the patient, determining that not all sensors are at a location external to the body of the patient; and
   in response to determining that not all sensors are at a location external to the body of the patient, generating a message indicating that not all sensors are at a location external to the body of the patient.
16. The method of paragraph 11, further comprising periodically receiving, at the first antenna of the surgical instrument, a low-power signal including identification and status information from the second antennas of the sensors.
17. The method of paragraph 11, further comprising receiving, at the first antenna, low-power EM signals, which include secure keys, that cannot be detected outside of the body of the patient to initiate secure communication between the surgical instrument and the sensors.
18. A surgical communication method, comprising:
   receiving, through a first antenna of a surgical instrument having a portion thereof disposed within a body of a patient, low-power EM signals, which include secure keys, that cannot be detected outside of the body of the patient to initiate secure communication between the surgical instrument and sensors placed within the body of the patient; and
   transmitting, from the first antenna of the surgical instrument, an EM signal to an instrumentation system disposed external to the body of the patient, the EM signal including EM sensor signals received by the first antenna from second antennas of the sensors.
19. The method of paragraph 18, further comprising:
   identifying sensors within the body of the patient;
   detecting movement of the surgical instrument to a location external to the body of the patient;
   in response to detecting movement of the surgical instrument to a location external to the body of the patient, determining that not all sensors are at a location external to the body of the patient; and
   in response to determining that not all sensors are at a location external to the body of the patient, generating a message indicating that not all sensors are at a location external to the body of the patient.
20. The method of paragraph 18, further comprising building a mesh network of the surgical instrument and the sensors.

## Claims

1. A surgical system, comprising:
an instrumentation system disposed external to a body of a patient;
sensors configured to be placed within the body of the patient, each sensor including a first communication device including a first antenna; and
a surgical instrument including a second communication device including a second antenna disposed at a proximal portion of the surgical instrument, the second communication device configured to communicate with the sensors via the first and second antennas, the second communication device further configured to communicate messages to or from the instrumentation system when the surgical instrument is placed in the body of the patient, the messages including sensor data from the sensors.

2. The surgical system of claim 1, wherein the second antenna is configured to act as a master antenna; and/or wherein the sensors are configured to communicate with each other via the first antennas of the sensors; preferably wherein the surgical instrument is a laparoscope.

3. The surgical system of any preceding claim, wherein the second communication device includes a microcontroller that configures the sensors to only communicate with the second communication device.

4. The surgical system of any preceding claim, further comprising a second surgical instrument including a third communication device, which includes a third antenna and a second microcontroller, the second microcontroller configured to communicate with the second communication device and the sensors via- the first, second, and third antennas.

5. The surgical system of any preceding claim, wherein the surgical instrument includes a microcontroller configured to build a mesh network including the surgical instrument and the sensors preferably wherein the instrumentation system includes a microcontroller configured to:
detect a failure event associated with the surgical instrument or one of the sensors; and
in response to detecting the failure event associated with the surgical instrument or one of the sensors, removing the surgical instrument or one of the sensors from the mesh network.

6. The surgical system of claim 5, wherein the failure event includes movement external to the body of the patient and/or wherein the failure event includes failure of a battery of at least one of the sensors.

7. A surgical communication method, comprising:
receiving, through a first antenna of a surgical instrument having a portion thereof disposed within a body of a patient, EM sensor signals from second antennas of sensors placed within the body of the patient; and
transmitting, from the first antenna of the surgical instrument, an EM signal to an instrumentation system disposed external to the body of the patient.

8. The method of claim 7, further comprising building a mesh network of first communication devices of the sensors and a second communication device of the surgical instrument.

9. The method of claim 7 or claim 8, further comprising:
detecting a termination event associated with the surgical instrument or one of the sensors; and
in response to detecting the termination event associated with the surgical instrument or one of the sensors, removing the second communication device of the surgical instrument or the first communication device of one of the sensors from the mesh network.

10. The method of claim 9, wherein detecting the termination event includes detecting movement of one of the sensors to a location external to the body of the patient or detecting failure of a battery of at least one of the sensors.

11. The method of claim 10, further comprising:
identifying sensors within the body of the patient;
detecting movement of the surgical instrument to a location external to the body of the patient;
in response to detecting movement of the surgical instrument to a location external to the body of the patient, determining that not all sensors are at a location external to the body of the patient; and
in response to determining that not all sensors are at a location external to the body of the patient, generating a message indicating that not all sensors are at a location external to the body of the patient.

12. The method of any of claims 7 to 11, further comprising periodically receiving, at the first antenna of the surgical instrument, a low-power signal including identification and status information from the second antennas of the sensors.

13. The method of any of claims 7 to 12, further comprising receiving, at the first antenna, low-power EM signals, which include secure keys, that cannot be detected outside of the body of the patient to initiate secure communication between the surgical instrument and the sensors.

14. A surgical communication method, comprising:
receiving, through a first antenna of a surgical instrument having a portion thereof disposed within a body of a patient, low-power EM signals, which include secure keys, that cannot be detected outside of the body of the patient to initiate secure communication between the surgical instrument and sensors placed within the body of the patient; and
transmitting, from the first antenna of the surgical instrument, an EM signal to an instrumentation system disposed external to the body of the patient, the EM signal including EM sensor signals received by the first antenna from second antennas of the sensors.

15. The method of claim 14, further comprising:
identifying sensors within the body of the patient;
detecting movement of the surgical instrument to a location external to the body of the patient;
in response to detecting movement of the surgical instrument to a location external to the body of the patient, determining that not all sensors are at a location external to the body of the patient; and
in response to determining that not all sensors are at a location external to the body of the patient, generating a message indicating that not all sensors are at a location external to the body of the patient, preferably still further comprising building a mesh network of the surgical instrument and the sensors.
